# EUROPEAN PATENT APPLICATION

(11) **EP 4 257 072 A1**
(43) Date of publication of application: **11.10.2023**
(21) Application number: 21899879.7
(22) Date of filing: 18.11.2021
(51) Int. Cl.: A61B 34/37

(54) **ROBOT ARM CONTROL METHOD AND LAPAROSCOPIC SURGICAL ROBOTIC SYSTEM**

(30) Priority: 04.12.2020 CN 202011408998
(71) Applicant: Harbin Intelligent Surgery Equipment Co., Ltd., Harbin, Heilongjiang 150000 (CN)
(72) Inventor: ZHANG, Jiaxing, Harbin, Heilongjiang 150000 (CN); WANG, Jianguo, Harbin, Heilongjiang 150000 (CN)
(74) Representative: Zaboliene, Reda
(86) International application number: PCT/CN2021/131421
(87) International publication number: WO 2022/116845

(57) **Abstract**

The present invention provides a robot arm control method and a laparoscopic surgical robotic system, in particular to the field of medical instruments. The laparoscopic surgical robotic system comprises an arm system and a plurality of consoles, wherein the arm system comprises a cavity endoscope arm and a plurality of surgical instrument arms, the cavity endoscope arm is adapted to mount a cavity mirror or a surgical instrument, and the surgical instrument arms are adapted to mount a surgical instrument or a cavity mirror; a plurality of the consoles are adapted to collectively control the endoscope arm and/or the surgical instrument arms. A function to shorten the surgery time is achieved by multiple consoles participating in the procedure simultaneously.

## Description

### Technical Field

The present invention relates to the field of medical instruments, and more particularly, to a robot arm control method and a laparoscopic surgical robotic system.

### Background Art

At present, a laparoscopic surgical robotic system is mainly composed of a surgical arm system with three arms or four arms and a mode in combination with one or two doctor consoles. A system of one console can only be operated by a doctor operating a surgical robot; the system of two doctor consoles works like this, i.e., when one doctor's operation stops, the other doctor' s console can operate, and two doctor consoles cannot participate in the surgery at the same time.

In case of emergency surgery, even several doctors cannot give a surgery at the same time, and the surgery cannot be completed more efficiently. At the same time, there is a great work load for the doctor giving the surgery. In addition, even with conventional surgery, it would be advantageous for a patient's postoperative recovery if the surgery time can be shortened.

### Summary of the Invention

A problem to be solved by the present invention is that the existing laparoscopic surgical robotic system is time-consuming in a surgery and the work load of one doctor is great.

In order to solve the above problems, the present invention provides a laparoscopic surgical robotic system comprising:
an arm system comprising an endoscope arm and a plurality of surgical instrument arms, wherein the endoscope arm is adapted to mount an endoscope or a surgical instrument, and the surgical instrument arms are adapted to mount a surgical instrument or an endoscope;
a plurality of consoles adapted to collectively control the endoscope arm and/or the surgical instrument arms.

Alternatively, the console comprises a console body and two primary operating hands and a foot pedal provided on the console body, the two primary operating hands correspond to a left-hand position and a right-hand position, respectively, the foot pedal corresponds to a foot position.

Alternatively, the console further comprises a touch control display apparatus adapted to receive operation permissions for the endoscope arm and the different surgical instrument arms.

In addition, the present invention also provides a robot arm control method based on the foregoing laparoscopic surgical robotic system, and the robot arm control method comprises:
learning the number of surgical instrument arms in the laparoscopic surgical robotic system; and
determining a scheme for assigning operation permissions for the endoscope arm and the surgical instrument arms according to the number of the surgical instrument arms, and transmitting the assignment to each of the consoles, such that different consoles jointly control the endoscope arm and/or the surgical instrument arms according to the assignment, wherein different consoles are adapted to simultaneously have operation permissions to control the endoscope arm.

Alternatively, the determining a scheme for assigning operation permissions for the endoscope arm and the surgical instrument arms according to the number of the surgical instrument arms comprises:
assigning, to a calibration console of the plurality of consoles operation permissions to control a portion of the plurality of surgical instrument arms, and assigning, to other consoles of the plurality of consoles operation permission to control another portion of the plurality of surgical instrument arms.

Alternatively, the determining a scheme for assigning operation permissions for the endoscope arm and the surgical instrument arms according to the number of the surgical instrument arms comprises:
if the number of surgical instrument arms is two, assigning, to the calibration console an operation permission to control two of the surgical instrument arms, or assigning, to the calibration console an operation permission to control one of the two surgical instrument arms and assigning, to the other console an operation permission to control the other of the two surgical instrument arms.

Alternatively, the determining a scheme for assigning operation permissions for the endoscope arm and the surgical instrument arms according to the number of the surgical instrument arms comprises:
if the number of the surgical instrument arms is three, assigning, to the calibration console an operation permission to control three of the surgical instrument arms, or assigning, to the calibration console an operation permission to control two of three of the surgical instrument arms and assigning, to the other consoles an operation permission to control another of three of the surgical instrument arms.

Alternatively, the determining a scheme for assigning operation permissions for the endoscope arm and the surgical instrument arms according to the number of the surgical instrument arms comprises:
if the number of the surgical instrument arms is four, assigning, to the calibration console an operation permission to control the four the surgical instrument arms, or assigning, to the calibration console an operation permission to control three of the four the surgical instrument arms, assigning, to the other console a control permission to control another of the four the surgical instrument arms, or assigning, to the calibration console an operation permission to control two of the four the surgical instrument arms, and assigning, to the other consoles an operation permission to control another two of the four surgical instrument arms.

Alternatively, each of the consoles obtains at most an operation permission for controlling two of the surgical instrument arms.

Alternatively, the controlling the endoscope arm and/or the surgical instrument arms comprises:
controlling movement of the surgical instrument arms via a primary operating hand in the console; and
controlling the movement of the endoscope arm by a foot pedal in the console and the primary operating hand.

Compared with the prior art, the robot arm control method and the laparoscopic surgical robotic system provided by the present invention have the following technical effects, but are not limited to:
determining, according to the number of specific robot arms (the total number of endoscope arms and surgical instrument arms), the assignment scheme of the operation permission of these robot arms in multiple consoles to ensure that the work load of the doctor on each console is minimized, and at the same time, multiple consoles can simultaneously manipulate the corresponding surgical instrument arms or/and endoscope arms of the console, and simultaneously participating in the surgery via multiple consoles to reduce the surgery time, which is extremely important for shortening the surgery time in emergent surgeries, and in addition, even in traditional surgeries, it would also be advantageous to be able to shorten the surgery time for the patient's postoperative recovery.

### Brief Description of the Drawings

FIG. 1 is a schematic flow diagram of a robot arm control method according to an embodiment of the present invention;
FIG. 2 is a schematic block diagram of a laparoscopic surgical robotic system according to an embodiment of the present invention.

### Description of Reference Numerals:

1-arm system, 11-surgical instrument arm, 12-endoscope arm, 2-console, 21-touch control display apparatus, 22-primary operating hand, 23-foot pedal.

### Detailed Description of the Invention

To make the above objects, features and advantages of the present invention more apparent, a detailed description of specific embodiments of the present invention will be made with reference to the accompanying drawings.

In describing the present invention, it should be noted that the terms "upper" and "lower" and the like designate orientations or positional relationships based on the orientation or positional relationships shown in the drawings, are merely for convenience in describing the invention and to simplify the description, and do not indicate or imply that the referenced devices or elements must have a particular orientation, be constructed and operated in a particular orientation, and thus should not be construed as limiting the invention.

Furthermore, in the figures, the Z axis represents vertical, i.e. up and down, the positive direction of the Z axis (i.e. the arrow of the Z axis points) represents up, and the negative direction of the Z axis (i.e. the direction opposite to the positive direction of the Z axis) represents down; it should also be noted that the foregoing Z-axis representation is merely intended to facilitate and simplify the description of the present invention and is not intended to indicate or imply that the device or element being referred to must have a particular orientation, be constructed and operated in a particular orientation and should not be construed as limiting the present invention.

The terms "first" and "second" etc. are used for descriptive purposes only and are not to be construed as indicating or implying relative importance or implicitly indicating the number of technical features indicated. Thus, features defining "first" and "second" may explicitly or implicitly include at least one such feature.

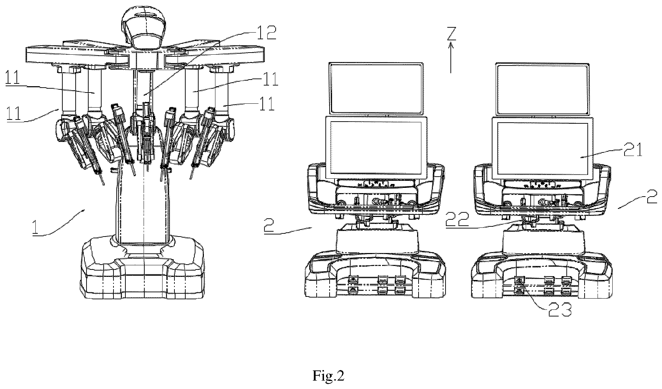

Referring to FIG. 2, an embodiment of the present invention provides a laparoscopic surgical robotic system including an arm system 1 and a plurality of consoles 2.

The arm system 1 comprises a cavity endoscope arm 12 and a plurality of surgical instrument arms 11, wherein the cavity endoscope arm 12 is adapted to mount an endoscope, and the surgical instrument arms 11 are adapted to mount a surgical instrument; a plurality of consoles 2 are adapted to jointly controlling the endoscope arm 12 and/or the surgical instrument arms 11.

It should be noted that in this embodiment, both the endoscope arm 12 and the surgical instrument arms 11 are robot arms with multiple degrees of freedom, which can be called robot arms, and the structures thereof can also be the same, but since the present system is applied to laparoscopic operation, there must be at least one robot arm mounted with an endoscope to provide a field of view during operation, and the robot arm mounted with the endoscope is denoted as the endoscope arm 12 for distinguishing several other robot arms mounted with surgical instruments, and the robot arm mounted with surgical instruments is denoted as the surgical instrument arms 11, and the endoscope arm 12 and the surgical instrument arms 11 belong to the arm system 1. It will be appreciated that the arm system may be referred to as a five-arm system if the sum of the endoscope arm 12 and the surgical instrument arms 11 is five, a four-arm system if the sum of the endoscope arm 12 and the surgical instrument arms 11 is four, and a three-arm system if the sum of the endoscope arm 12 and the surgical instrument arms 11 is three.

Referring to FIG. 2, preferably, the console 2 includes a console body and two primary operating hands 22 and a foot pedal 23 provided on the console body, wherein the two primary operating hands 22 correspond to a left-hand position and a right-hand position, respectively, and the foot pedal 23 corresponds to a foot position.

Preferably, the console further comprises a touch control display apparatus 21 adapted to receive operation permissions for the endoscope arm 12 and the different surgical instrument arms 11. That is, the user can input the operation permission for the different robot arms through the touch control display apparatus 21.

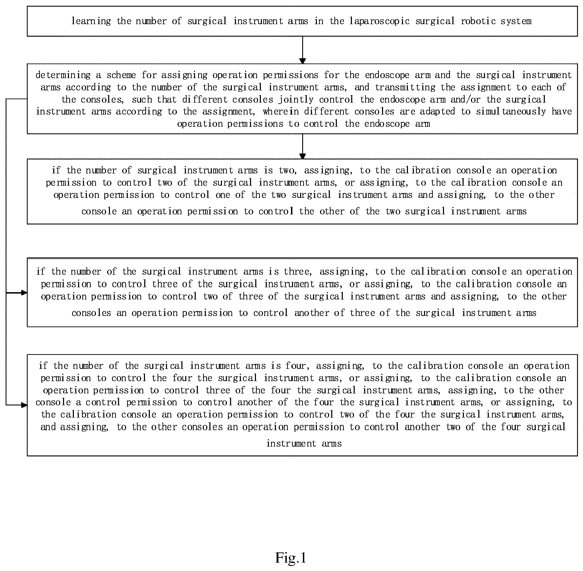

Referring to FIG. 1, in addition, another embodiment of the present invention provides a robot arm control method based on the foregoing laparoscopic surgical robotic system, and the robot arm control method comprises:
learning the number of surgical instrument arms 11 in the laparoscopic surgical robotic system; and
determining a scheme for assigning operation permissions of the endoscope arm 12 and the surgical instrument arms 11 according to the number of surgical instrument arms 11, and transmitting the assignment scheme to each console 2, so that different consoles 2 control the endoscope arm 12 and/or the surgical instrument arms 11 together according to the assignment scheme, wherein the different consoles 2 are adapted to simultaneously have the operation permission to control the endoscope arm 12.

Here, this embodiment provides a robot arm control method for determining the operation permission assignment scheme of the robot arms in the plurality of consoles 2 according to the number of robot arms in the specifically selected arm system 1 to ensure that the work load of the doctor on each console 2 is minimized, and at the same time, the plurality of consoles 2 can simultaneously operate the corresponding surgical instrument arms 11 or/and the endoscope arm 12 of the console 2 to simultaneously participate in the surgery through the plurality of consoles 2, thereby reducing the surgery time, which is of great significance for shortening the surgery time in the case of emergent surgeries, in addition, even in the case of conventional surgeries, it would also be advantageous to be able to shorten the surgery time for the patient's postoperative recovery.

Here, since each console may have the right to control the endoscope arm 12, when giving a surgery, it can be ensured that a doctor (denoted as the first doctor) on one console (denoted as the first console) performs the control of the surgical instrument arms 11 on the first console, and a doctor (denoted as the second doctor) on the other console (denoted as the second console) performs the control of the endoscope arm 12 on the second console to provide the first doctor with the best view, although the control of the endoscope arm 12 may also be controlled by the first doctor on the first console.

It can be understood that two robot arms for mounting a cavity mirror can also be used, for example, in a five-arm system, two robot arms are used for mounting a cavity mirror, and the other three robot arms are used for mounting a surgical instrument; furthermore, two robot arms for mounting a cavity mirror are called a cavity endoscope arm 12, and three robot arms for mounting a surgical instrument are called surgical instrument arms 11, that is to say, in the case of a certain number of robot arms, when the number of robot arms for mounting a cavity mirror increases by one, the number of robot arms for mounting a surgical instrument correspondingly decreases by one, and two cavity endoscope arms 12 can simultaneously participate in a surgery. As to the assignment mode of the operation permission of the second endoscope arm, the second endoscope arm can be temporarily classified into the surgical instrument arms 11, and after the first endoscope arm and all the surgical instrument arms have been assigned, an endoscope is mounted on a certain surgical instrument arms 11 to be used as the second endoscope arm according to specific situations.

It should be noted that the term "operation permission" in this embodiment means that the console 2 has the right to control a certain robot arm (the endoscope arm 12 may be called a robot arm, and the surgical instrument arms 11 may also be called a robot arm) or a certain robot arm, and the console 2 can only control the movement of the robot arm after having the right to control the specific robot arm. In other words, it is not necessary for the robot arm to be controlled with a certain robot arm's operation permission, and it is possible to choose to operate at a certain time, a certain demand, for example, according to actual situations, but it is impossible to control the robot arm without the robot arm' s operation permission.

Preferably, the determining a scheme for assigning operation permissions for the endoscope arm 12 and the surgical instrument arms 11 according to the number of the surgical instrument arms 11 comprises:
assigning, to a calibration console of the plurality of consoles 2 operation permissions to control a portion of the plurality of surgical instrument arms 11, and assigning, to other consoles of the plurality of consoles 2 operation permission to control another portion of the plurality of surgical instrument arms 11.

Here, it is to be noted that the above-mentioned robot arm control method can be implemented by an external server or other device, i.e., knowing the number of surgical instrument arms 11 in the laparoscopic surgical robotic system via the external server or other device, determining an assignment scheme of the operation permission of the endoscope arm 12 and the surgical instrument arms 11 according to the number of surgical instrument arms 11, and then transmitting the assignment scheme to each console 2; an external server or other devices may then automatically assign operation permissions to control a portion of the plurality of surgical instrument arms 11 to the calibration console according to the assignment scheme, while automatically assigning operation permission to control another portion of the plurality of surgical instrument arms 11 to other consoles. Of course, it is also possible to manually select a calibration console from the touch control display apparatus 21 of the console by the doctor after communicating with each other, the other consoles having operation permission for controlling a certain surgical instrument arms or arms. In general, the operation permission of the surgical instrument arms by the console 2 can be obtained automatically or manually by a doctor. Of course, each console may not be the aforementioned "the different consoles 2 are adapted to simultaneously have the operation permission to control the endoscope arm 12", or the operation permission for controlling the cavity endoscope arm 12 by one or some of the consoles 2 may be manually canceled in the touch control display apparatus 21.

With reference to FIG. 1, the determining a scheme for assigning operation permissions for the endoscope arm 12 and the surgical instrument arms 11 according to the number of the surgical instrument arms 11 comprises:
if the number of surgical instrument arms 11 is two, assigning, to the calibration console an operation permission to control two of the surgical instrument arms 11, or assigning, to the calibration console an operation permission to control one of the two surgical instrument arms 11 and assigning, to the other console an operation permission to control the other of the two surgical instrument arms 11.

Here, two surgical instrument arms 11 plus one endoscope arm 12, i.e., for a three-arm system, the operation permission of the two surgical instrument arms 11 (i.e., a first surgical instrument arms and a second surgical instrument arm) can be assigned to one console (i.e., a first console), and when the other console (a second console) also has the operation permission of the endoscope arm 12, so that a doctor (i.e., a first doctor) can manipulate the first surgical instrument arms and the second surgical instrument arms on the first console to perform a surgical operation when performing a surgical operation, another doctor (referred to as a second doctor) can manipulate the endoscope arm 12 on the second console to provide the first doctor with an optimal field of view, which can greatly reduce the work load of the first doctor and, at the same time, can shorten the surgery time. Of course, it is also possible to have the first console and the second console each have the control permission of one surgical instrument arms 11.

Here, the first doctor may be a primary doctor and the second doctor may be a first assistant. For learning operation, the first assistant can learn the surgical steps, points, etc. more actively and intuitively by actually operating the robot arms.

With reference to FIG. 1, the determining a scheme for assigning operation permissions for the endoscope arm 12 and the surgical instrument arms 11 according to the number of the surgical instrument arms 11 comprises:
if the number of the surgical instrument arms 11 is three, assigning, to the calibration console an operation permission to control three of the surgical instrument arms 11, or assigning, to the calibration console an operation permission to control two of three of the surgical instrument arms 11 and assigning, to the other consoles an operation permission to control another of three of the surgical instrument arms 11.

With reference to FIG. 1, the determining a scheme for assigning operation permissions for the endoscope arm 12 and the surgical instrument arms 11 according to the number of the surgical instrument arms 11 comprises:
if the number of the surgical instrument arms 11 is four, assigning, to the calibration console an operation permission to control the four the surgical instrument arms 11, or assigning, to the calibration console an operation permission to control three of the four the surgical instrument arms 11, assigning, to the other console a control permission to control another of the four the surgical instrument arms 11, or assigning, to the calibration console an operation permission to control two of the four the surgical instrument arms 11, and assigning, to the other consoles an operation permission to control another two of the four surgical instrument arms 11.

For a difficult operation, more robot arms can help the doctor complete the complicated and accurate operation, reduce the blood loss, shorten the complicated surgery time and ensure patient safety.

Preferably, the controlling the endoscope arm 12 and/or the surgical instrument arms 11 comprises:
controlling the movement of the surgical instrument arms 11 via a primary operating hand 22 in the console 2; and
controlling the movement of the endoscope arm 12 by a foot pedal 23 and a primary operating hand 22 in the console 2.

Here, when it is required to move the endoscope arm 12, the foot pedal 23 is pressed firstly, and then the left hand and the right hand simultaneously operate the two primary operating hands 22 to perform the same movement to move the endoscope arm 12, and after releasing the foot pedal 23, the endoscope arm 12 stops moving.

Preferably, each console 2 obtains at most the operation permission to control the two surgical instrument arms 11.

Here, on the same console 2, at most two primary operating hands 22 can be operated simultaneously by both hands of a doctor, and at most two surgical instrument arms 11 can be controlled simultaneously by one doctor, so that each console 2 has at most operation permission to control both surgical instrument arms 11.

In addition, it needs to be stated that if the existing laparoscopic surgical robots have failures in the surgery, they can only be removed at once, a surgery by the robot is interrupted and other rescue measures are taken, which may bring great risks to the surgery and reduce the surgical effect. However, in the arm system and the corresponding control method provided in this embodiment, when the configuration has four or more surgical instrument arms 11 and two or more control consoles 2, when the surgical instrument arms 11 or the control consoles 2 fail, as long as two or more robot arms and one or more control consoles 2 in the arm system 1 are normal, the robotic surgery can be completed, and the risk of surgery interruption due to equipment failure is greatly reduced.

Although the present disclosure has been described above, the endoscope of protection of the present disclosure is not limited thereto. Various changes and modifications may be affected by a person skilled in the art without departing from the spirit and endoscope of the disclosure, and it is intended that such changes and modifications fall within the endoscope of the appended claims.

## Claims

1. A laparoscopic surgical robotic system, comprising:
an arm system (1) comprising an endoscope arm (12) and a plurality of surgical instrument arms (11), wherein the endoscope arm (12) is adapted to mount an endoscope or a surgical instrument (11), and the surgical instrument arms (11) are adapted to mount a surgical instrument or an endoscope;
a plurality of consoles (2) adapted to collectively control the endoscope arm (12) and/or the surgical instrument arms (11).

2. The laparoscopic surgical robotic system according to claim 1, wherein the console (2) comprises a console body and two primary operating hands (22) and a foot pedal (23) provided on the console body, the two primary operating hands (22) correspond to a left-hand position and a right-hand position, respectively, and the foot pedal (23) corresponds to a foot position.

3. The laparoscopic surgical robotic system according to claim 1, wherein the console comprises a touch control display apparatus (21) adapted to receive operation permissions for the endoscope arm (12) and different surgical instrument arms (11).

4. A robot arm control method based on the laparoscopic surgical robotic system according to any one of claims 1 to 3, wherein the robot arm control method comprises:
learning the number of surgical instrument arms (11) in the laparoscopic surgical robotic system; and
determining a scheme for assigning operation permissions for the endoscope arm (12) and the surgical instrument arms (11) according to the number of the surgical instrument arms (11), and transmitting the assignment to each of the consoles (2), such that different consoles (2) jointly control the endoscope arm (12) and/or the surgical instrument arms (11) according to the assignment, wherein different consoles (2) are adapted to simultaneously have operation permissions to control the endoscope arm (12).

5. The robot arm control method according to claim 4, wherein the determining a scheme for assigning operation permissions of the endoscope arm (12) and the surgical instrument arms (11) according to the number of surgical instrument arms (11) comprises:
assigning, to a calibration console of the plurality of consoles (2) operation permissions to control a portion of the plurality of surgical instrument arms (11), and assigning, to other consoles of the plurality of consoles (2) operation permission to control another portion of the plurality of surgical instrument arms (11).

6. The robot arm control method according to claim 5, wherein the determining a scheme for assigning operation permissions of the endoscope arm (12) and the surgical instrument arms (11) according to the number of surgical instrument arms (11) comprises:
if the number of surgical instrument arms (11) is two, assigning, to the calibration console an operation permission to control two of the surgical instrument arms (11), or assigning, to the calibration console an operation permission to control one of the two surgical instrument arms (11) and assigning, to the other console an operation permission to control the other of the two surgical instrument arms (11).

7. The robot arm control method according to claim 5, wherein the determining a scheme for assigning operation permissions of the endoscope arm (12) and the surgical instrument arms (11) according to the number of surgical instrument arms (11) comprises:
if the number of the surgical instrument arms (11) is three, assigning, to the calibration console an operation permission to control three of the surgical instrument arms (11), or assigning, to the calibration console an operation permission to control two of three of the surgical instrument arms (11) and assigning, to the other consoles an operation permission to control another of three of the surgical instrument arms (11).

8. The robot arm control method according to claim 5, wherein the determining a scheme for assigning operation permissions of the endoscope arm (12) and the surgical instrument arms (11) according to the number of surgical instrument arms (11) comprises:
if the number of the surgical instrument arms (11) is four, assigning, to the calibration console an operation permission to control the four the surgical instrument arms (11), or assigning, to the calibration console an operation permission to control three of the four the surgical instrument arms (11), assigning, to the other console a control permission to control another of the four the surgical instrument arms (11), or assigning, to the calibration console an operation permission to control two of the four the surgical instrument arms (11), and assigning, to the other consoles an operation permission to control another two of the four surgical instrument arms (11).

9. The robot arm control method according to claim 5, wherein each of the consoles (2) obtains at most an operation permission for controlling two surgical instrument arms (11).

10. The robot arm control method according to claim 4, wherein the controlling the endoscope arm (12) and/or the surgical instrument arms (11) comprises:
controlling movement of the surgical instrument arms (11) via a primary operating hand (22) in the console (2); and
controlling the movement of the endoscope arm (12) by a foot pedal (23) in the console (2) and the primary operating hand (22).
